# EUROPEAN PATENT APPLICATION

(11) **EP 4 431 118 A1**
(43) Date of publication of application: **18.09.2024**
(21) Application number: 23305931.0
(22) Date of filing: 12.06.2023
(51) Int. Cl.: A61K 51/08, A61P 35/00

(54) **METHOD FOR EARLY PREDICTION OF THE EFFICACY OF PRRT IN PATIENTS WITH METASTATIC NEUROENDOCRINE TUMOURS**

(30) Priority: 14.03.2023 EP 23305344
(71) Applicant: Assistance Publique Hôpitaux de Paris, 75012 Paris (FR)
(72) Inventor: DE MESTIER DU BOURG, Louis, 92110 Clichy (FR); LEBTAHI, Rachida, 92110 Clichy (FR); BANDO-DELAUNAY, Aurélie, 92110 Clichy (FR); RUSZNIEWSKI, Philippe, 92110 Clichy (FR)
(74) Representative: Novagraaf Technologies

(57) **Abstract**

The present invention relates to an ex *vivo* method for predicting peptide-receptor radionuclide therapy (PRRT) efficacy in a patient with a malignant tumor, comprising the steps of:
a) Measuring the radioactivity emitted by radionuclides after a first cycle (C1) and a second cycle (C2) of PRRT to the patient, wherein C2 is performed less than 9 months after C1, and
b) comparing the radioactivity at C1 and the radioactivity at C2,
Wherein:
• If the radioactivity at C2 is inferior to the radioactivity at C1, the complete PRRT is considered likely to have an efficacy for the patient, or
• If the radioactivity at C2 is superior to the radioactivity at C1, the complete PRRT is considered unlikely to have an efficacy for the patient.

## Description

### Technical field

The present invention refers to an ex *vivo* method for predicting the efficacy of peptide-receptor radionuclide therapy (PRRT) in a patient with a malignant tumor.

Therefore, the present invention has utility in the medical field, especially in the field of predictive medicine.

In the description below, the references into brackets ([ ]) refer to the listing of references situated at the end of the text.

### Background of the Invention

Neuroendocrine tumors (NETs) are relatively rare neoplasms with increasing incidence. Approximately half of NETs are associated with metastases, notably those arising from the gastro-intestinal tract or the pancreas. Due to the usually slow growth of NETs, patients generally have prolonged survival even in case of metastases, with a median survival of approximately 5 years.

During the last decade, the peptide-receptor radionuclide therapy (PRRT) targeting somatostatin receptors using ¹⁷⁷Lu-DOTATATE has gained a major place in the therapeutic strategy of patients with metastatic NETs showing strong expression of somatostatin receptors (Pavel *et al.* 2016, 2020 ([1]); de Mestier *et al.* 2020 ([2])). PRRT generally consists in four cycles of 7,4 GBq, administered every 8-10 weeks. In patients with progressive metastatic gastro-intestinal NETs, the NETTER-1 phase 3 trial showed that ¹⁷⁷Lu-DOTATATE combined with long-acting octreotide significantly increased progression-free survival (PFS) in comparison with double-dose octreotide (28.4 vs. 8.5 months, respectively; HR 0.21, 95% CI [0.14-0.33], p<0.0001), and provided higher objective response rate (18% vs. 3%, respectively, p<0.001).

Objective tumour response is usually measured after the completion of PRRT, by comparing contrast-enhanced computed tomography (CT) scans performed at baseline and 3-6 months after the fourth PRRT cycle according to the RECIST 1.1 criteria (Eisenhauer *et al.* 2009 ([3])). PRRT implies a long duration between the first and the last cycle (usually 6 months), and an even longer duration between the baseline and the evaluation CT scans (9-12 months). To date, no clinical, biological or imaging marker exists for the early prediction of PRRT efficacy. Such early marker could help identifying without delay the patients who might benefit from treatment change or optimisation.

Thus, an unmet need exists regarding methods for early prediction of PRRT efficacy. The present invention fulfills these and other needs.

### Description of the invention

The Applicant has found surprisingly that the evolution of tumour uptake during successive cycles of PRRT can predict its efficacy.

Indeed, surprisingly, the results of their work lead to the conclusion that the evolution of the uptake of liver metastases on post-PRRT scintigraphy between cycle 1 (C1) and cycle 2 (C2), can be associated with tumour efficacy measured after complete PRRT cycle, i.e. usually cycle 4 (C4), in patients with metastatic NETs treated with ¹⁷⁷Lu-DOTATATE.

Therefore, the present invention provides an *ex vivo* method for predicting peptide-receptor radionuclide therapy (PRRT) efficacy in a patient with a malignant tumor, comprising the steps of:
a) Measuring tumor uptake after a first cycle (C1) and a second cycle (C2) of PRRT to the patient, and
b) comparing tumor uptake measured at C1 and tumor uptake measured at C2,
Wherein:
- If tumor uptake at C2 is inferior to tumor uptake at C1, the complete PRRT is considered likely to have an efficacy for the patient, or
- If tumor uptake at C2 is superior to tumor uptake at C1, the complete PRRT is considered unlikely to have an efficacy for the patient.

"Malignant tumor" refers herein to any neoplasm likely to be treated by PRRT. It may be for example, but not limited to, neuroendocrine neoplasms, prostate neoplasms, breast neoplasms, liver neoplasms, or pancreatic neoplasms. Neuroendocrine neoplasms include well-differentiated neuroendocrine tumors (NET) from digestive or other/unknown origin, classified as grade 1, 2 or 3 according to the 2019 WHO classification. Especially, a "malignant neoplasm" may be a metastatic neoplasm, as are metastatic small-bowel or pancreatic grade 1 or grade 2 NETs.

"Peptide receptor radionuclide therapy (PRRT)" refers herein to a type of radionuclide therapy, using a radiopharmaceutical compound that targets peptide receptors to deliver localized internal radiotherapy, typically for NETs. PRRT allows the targeted delivery of therapeutic radionuclides directly to the target site, as it targets somatostatin receptors in NETs. A radioactive substance is combined with a relevant peptide or its analogue, usually a somatostatin analogue in NETs, so that it preferentially binds to the neoplasm that express the target. It can be for example analogue materials such as octreotide and other DOTA (2,2',2",2‴-(1,4,7,10-Tetraazacyclododecane-1,4,7,10-tetrayl)tetraacetic acid) compounds, which may be combined with lutetium-177 or yttrium-90 for example. For example, the PRRT compound may include, but not limited to, ¹⁷⁷Lu-DOTA-peptide (¹⁷⁷Lu-DOTATATE, ¹⁷⁷Lu-DOTATOC), ¹⁷⁷Lu-PSMA, ²²⁵Ac-DOTA-peptide and ⁹⁰Y-DOTA-peptide. The ¹⁷⁷Lutetium radioisotope, or other radioisotopes used for PRRT, emits β particles but also γ rays detectable on scintigraphy. Hence, post-therapeutic scintigraphy is usually performed following each PRRT administration in order to verify the appropriate targeting of the different localizations of the malignant neoplasm.

"Tumor uptake" refers herein to the amount of radiopharmaceutical compound visible on imaging that is taken up by the cancerous organ(s).

"Cycle" of PRRT refers herein to the administration of a dose of PRRT, also referenced to as radionuclides, to a patient. Each cycle of PRRT may be advantageously the administration of the same dose of PRRT to a patient, or of different doses. Advantageously, each cycle of PRRT, especially C1 and C2, may be the administration of the same dose of PRRT to a patient. A complete PRRT may comprise at least 2 cycles, or at least 3, or at least 4, or at least 5, or at least 6, or more, that may be referenced to as C1, C2, C3, C4, C5, C6, etc..., Cn. Usually, a complete PRRT usually comprises 4 cycles, especially in neuroendocrine tumors). A lower number of PRRT cycles may be administered in case of early progression or unacceptable toxicity. Also, a superior number of PRRT may be administered if needed, depending on the patient's needs, the type of malignant neoplasm and the type of radionuclide. These cases do not modify the embodiment of the invention. A complete cycle of PRRT may last about 6 months to about 24 months usually about 8 or 9 months in NETs, from the C1 to the evaluation post-C4.

Therefore, "first cycle" or C1, refers herein to the administration of a first dose of PRRT to the patient, and "second cycle" or C2 refers herein to the administration of a next dose of PRRT to the patient. Advantageously, the first cycle and the second cycle may be spaced by about 6 weeks to 4 months, for example usually between 6 weeks and 10 weeks in NETs. Advantageously, C2 is performed less than 9 months after C1, or less than 8 months, or less than 7 months, or less than 6 months, or less than 5 months, or less than 4 months, or less than 3 months. Advantageously, C2 may be performed about 1 month, or 2 months, or 3 months, or 4 months (about 8 weeks) after C1.

As the radioisotope used for PRRT emits γ rays, post-therapeutic nuclear imaging, such as a scintigraphy technique, advantageously using a gamma camera, can detect all tumor and physiological uptake of any PRRT injection. Measurement of the radioactivity emitted by radionuclides, or the tumoral uptake, can be obtained by any technique known in the art, for example by measuring the count rate using the Syngo^{®} (Siemens Healthcare^{™}) software, used for any type of acquisition, for example, 2D or 3D, planar anterior or posterior, whole-body or segmental, and any type of arithmetic transformation, for example using the geometric mean of both anterior and posterior images after C1 and C2 cycles. Advantageously, the measurement on post-therapeutic imaging, e.g., scintigraphy, may be acquired at any suitable time after PRRT cycle, for example between about 1 hour and 96 hours after PRRT administration. For example, the measurement may be performed between about 1 and 24 hours after PRRT administration, or about 4 and about 24 hours after PRRT administration or about 4 and about 21 hours after PRRT administration so that the quality of images is optimal. Advantageously, the measurement may be performed to obtain an anterior and posterior acquisition, which allows for a geometric mean of the radioactivity emitted by radionuclides after C1 and C2.

The measurement may be performed on the zone targeted by the PRRT. Advantageously, it may consists in the primary tumor, in metastases or in both. It may be advantageously on metastases only. For example, metastases may be liver metastases, as they are the most frequent metastatic dissemination site in patients with NETs (>95%), and easily reproducible.

"C2/C1 ratio" refers herein to the ratio between the result of the measurement of the radioactivity emitted by radionuclides (for example geometric mean of the counts on anterior and posterior views) after the second cycle (C2) and the result of the measurement of the radioactivity emitted by radionuclides after the first cycle (C1). Advantageously, this ratio reflects the variation of the tumor uptake of radionuclides between C1 or C2. Also, the variation of tumor uptake between C1 or C2 may be measured by other arithmetic transformations, which do not modify the embodiment of the invention.

"Efficacy" refers herein to at least one of the criteria chosen among morphological response, e.g., a decrease in the size, density and/or other measurable variable on imaging, for example a decrease superior or equal to 30% in the sum of target lesions according to RECIST 1.1 criteria, the biological response, the progression-free survival and the overall survival.

Advantageously, step a) may comprise the steps of:
a1) contouring the whole organ which includes the tumor on nuclear imaging, and
a2) measuring tumor uptake, above threshold of normal organ uptake.

Contouring may be carried out by any means known in the art, be it manually, automatically or semi-automatically.

"Threshold of normal organ uptake" refers herein to the threshold of uptake that the non-cancerous organ would have. This could be established by calculating the mean of this uptake on a statistically appropriated number of non-cancerous organ of the same type as the malignant organ.

Advantageously, step a) may comprise also the step of:
a3) calculating tumor uptake.

Advantageously, step b) may comprise the step of calculating the C2/C1 ratio of tumor uptake,
and wherein:
- If the C2/C1 ratio is < 0.85 or < 1.00, the complete PRRT is considered likely to have an efficacy for the patient, or
- If the C2/C1 ratio is ≥ 0.85 or ≥ 1.00, the complete PRRT is considered unlikely to have an efficacy for the patient.

Advantageously, if the C2/C1 ratio is < 1.00, the patient is considered likely to have an increased progression free survival (PFS) after a complete PRRT compared to a patient having a C2/C1 ratio ≥ 1.00. PFS is the length of time measured from the initiation of the treatment to the time of disease progression or death. In other words, PFS measures the duration of time during and after the treatment of a disease, during which a patient lives with the disease but it does not get worse.

### Brief description of the figures

- Figure 1: represents whole-body SPECT images performed 4 hours after PRRT injection, at C1 (A) and C2 (B), in a patient with a grade 1 metastatic neuroendocrine tumor.
- Figure 2: represents the flow chart of the study.
- Figure 3: represents the C2/C1 ratio and correlation with morphological objective response to PRRT. 3A represents the correlation between the 4-hour and the 21-hour C2/C1 ratios, when both 4-hour and 21-hour scintigraphies were performed at both C1 and C2. 3B represents the ROC curve of the association between the C2/C1 ratio and morphological objective response to PRRT. 3C represents thee comparison of the C2/C1 ratio between responders and non-responders to PRRT. 3D represents the comparison of the proportion of responders and non-responders to PRRT depending on the C2/C1 ratio.
- Figure 4: represents the progression-free survival actuarial curves depending on the C2/C1 ratio. 4A represents the comparison of cases with a C2/C1 ratio ≥ or < 0.85. 4B represents the comparison of cases with a C2/C1 SPECT ratio ≥ or < 1.

### EXAMPLE

**Purpose:** PRRT has a major place in the therapeutic strategy of metastatic NETs. Its efficacy is generally evaluated after four cycles, with no early predictor identified to date. During each PRRT cycle, ¹⁷⁷Lu-related gamma emissions can be measured by post-PRRT scintigraphy. We explored whether the variation in tumor uptake on post-PRRT scintigraphy performed at C1 and C2 could predict the efficacy of PRRT.

**Patients:** We studied all consecutive patients who received at least 2 cycles of PRRT from 2013 to 2019 for metastatic NET. We calculated the C2/C1 ratio of liver metastases uptake (geometric mean of anterior and posterior uptakes) on post-PRRT scintigraphy. We explored the association between the C2/C1 ratio and the RECIST-defined response measured after C4, and progression free survival (PFS).

### Patients and methods

### Patients

We performed a retrospective study of all consecutive patients with histologically proven well-differentiated NET of any grade who received at least 2 cycles of PRRT with ¹⁷⁷Lu-DOTATATE at Beaujon Hospital (ENETS Centre of Excellence, Clichy, France) between July 1^{st}, 2013 and December 31^{th}, 2019. Among them, we included all patients with liver metastases who performed post-PRRT scintigraphy at C1 and C2. We excluded patients without measurable liver targets and/or who received different doses of PRRT at C1 and C2. One patient could be included twice in case of two successive, distinct PRRT treatments, should the PRRT rechallenge be performed at least 12 months after the last cycle of the first PRRT sequence.

This study was performed according to the Helsinki convention. Data collection was anonymous following patient consent and institutional review board approval (CEERB Paris-Nord University, IRB 00006477-15-073).

### PRRT and post-PRRT scintigraphy

The decision of initiating PRRT was always made during NET-dedicated multidisciplinary tumour boards, based on RECIST-defined progression based on CT scans and intensity of somatostatin receptor imaging uptake (Krenning scale 2, 3 or 4) assessed on ¹¹¹In-pentetreotide scintigraphy (Octreoscan^{®}) or ⁶⁸Ga-DOTATOC PET/CT . Each PRRT cycle consisted in an intravenous infusion of 7.4 GBq (200 mCi) of ¹⁷⁷Lu-DOTATATE. The planned treatment consisted in four cycles, one every 8 weeks, unless unacceptable treatment-related adverse events or RECIST 1.1-defined disease progression, as confirmed by NET-dedicated multidisciplinary tumour boards. Dose reduction could be applied in case of treatment-related adverse events or high risk of toxicity (e.g., important bone metastatic burden and/or cytopenia prior to PRRT).

Whole-body scintigraphy was performed after PRRT administration at each cycle, using the same gamma-camera (Symbia T, Siemens Healthineers, Erlangen, Germany). Acquisition was performed in the supine position using medium energy parallel hole collimators with 20% energy window centred at photopeaks of 208 keV and 113 keV. Before 2019, post-PRRT scintigraphy was performed 21 hours or 4 hours after PRRT administration, or both. Since 2019, post-PRRT scintigraphy was only performed 4 hours after PRRT administration, because of the need to move toward outpatient management, notably favoured by the COVID-19 pandemic.

Routine clinical examinations and biology tests were performed before each PRRT cycle. CT scans were performed within 3 months before C1, between the C2 and C3, and then 3 months and 6 months after the last treatment and thereafter every 6 months, or whenever tumour progression was suspected by suggestive clinico-biological symptoms.

### Data collection

We retrospectively collected anonymized data on epidemiological, clinical and histopathological features and treatments received. All pre-PRRT pathological specimens were centrally reviewed by pathologists with expertise in NETs and were classified according to the 2019 WHO classification based on differentiation and grade (WHO Classification of Tumours. 2019).

All CT scans were centrally reviewed to identify the localization and number of metastases and measure the size of target lesions. The best morphological response was determined by comparing target and non-target lesions between the CT scan following the last PRRT cycle and CT-scan performed at baseline, according to RECIST 1.1 criteria (objective response, stable disease or progression) and the date of disease progression (Eisenhauer *et al.* 2009 ([3])).

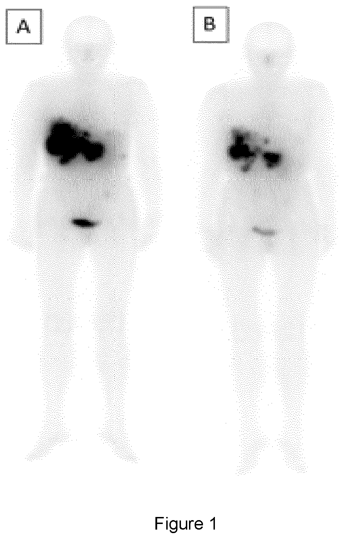

All scintigraphy imaging performed at C1 and C2 were centrally reviewed by two nuclear physicians. The liver region was delimited manually using the region-of-interest technique. We measured the count rate (kcps) using the Syngo^{®} (Version, developer) software on both anterior and posterior acquisitions (Figure 1). We computed the geometric mean of the count rates measured on anterior and posterior views at C1 and C2 and calculated the C2/C1 ratio.

### Statistical analyses

Continuous variables were described as medians and their 25-75 interquartile range (IQR) and were compared using the Mann-Whitney test. Qualitative variables were described as frequencies and percentages and were compared using the Chi-2 or the Fischer test, wherever most appropriate. The Spearman test was used to evaluate the correlation between the 4-hour and the 21-hour C2/C1 ratios, considering treatments for which both 4-hour and 21-hour scintigraphies were performed at both C1 and C2.

The primary endpoint was the objective response rate, as defined by RECIST 1.1 criteria on the CT scan following the last PRRT cycle. A receiver-operator curve (ROC) was computed to assess the relationship between the C2/C1 ratio and the objective response rate, using the area under the curve (AUC), and the best predictive threshold using the Youden index method. Factors associated with the probability of objective response were explored using a multivariate logistic regression backward-stepwise analysis, considering all non-collinear, clinically relevant variable with univariable p-value < 0.20 as input.

The secondary endpoint was PFS, defined as the time elapsed from the first PRRT cycle to the time of RECIST-defined progression or death; otherwise, patients were censored at their last follow-up. The point date was set on May 1, 2022. Median PFS (and their 95% confidence interval [CI]) were estimated using the Kaplan-Meier method and compared using the log-rank test. Factors associated with the risk of progression or death were explored using a multivariate Cox proportional hazard backward-stepwise analysis, considering all non-collinear, clinically relevant variable with univariable p-value < 0.20 as input. Of note, the concomitant administration of long-acting somatostatin analogues was forced into the model given of its hypothetical impact on PFS (Yordanova *et al.* 2018 ([4])).

A p-value <0.05 was considered to be statistically significant. The analyses were performed using Prism^{©} (version 6, Graphpad^{™}) and SPSS^{©} (version 20, IBM^{™}) softwares.

### Results

### Patients and treatments

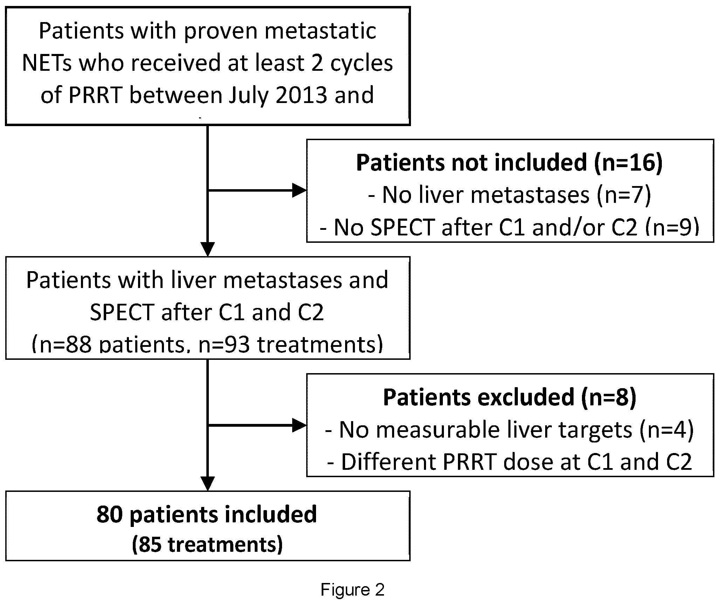

Among the 104 patients with proven metastatic NETs who received at least 2 cycles of PRRT between July 2013 and December 2019 in our institution, we did not include seven patients without liver metastases and nine patients without post-PRRT SPECT images available for review (Figure 2). We further excluded 8 additional patients without measurable liver targets or who received different PRRT dose between C1 and C2. The population corresponding to the 85 treatments was composed of 39 female and 46 male patients, of median age 65.4 years (Table 1). We included 80 patients (85 treatments received), with small-bowel (57%) or pancreatic (26%), G1 (31%) or G2 (65%) NET. Most NETs (56.5%) originated from the small intestine. A functioning syndrome accounted for 46 (54.1 %) of cases and mostly consisted in carcinoid syndrome. Liver metastatic involvement > 50% and/or extrahepatic metastases were noted in 44.7% and 92.9% of cases, respectively. All NETs were well differentiated and approximately two-thirds of them were classified as G2 or G3. Before PRRT initiation, patients had undergone primary tumour resection in 75.3% of cases and had received 2 different non-surgical treatment lines on average, the most frequent one being long-acting somatostatin analogues (84.7% of cases) (Table 1). All patients had documented progressive disease at PRRT initiation.

**Table 1. Characteristics of the patients corresponding to the 85 PRRT treatments**

| | |
|---|---|
| **Age (years), median (IQR)** | 65.4 (57-71) |
| **Female sex, n (%)** | 39 (45.9) |
| **Body mass index (kg/m²), median (IQR)** | 22.6 (20.8-25.5) |
| **Primary tumor origin, n (%)** | |
| Pancreas | 22 (25.9) |
| Small intestine | 48 (56.5) |
| Colon/rectum | 7 (8.2) |
| Other/unknown | 8 (9.4) |
| **Performance status, n (%)** (3 missing) | |
| PS-0 | 52 (63.4) |
| PS-1 or PS-2 | 30 (36.6) |
| **Functioning syndrome, n (%)** | 46 (54.1) |
| Carcinoid syndrome | 41 (48.2) |
| Other | 5 (5.9)* |
| **Carcinoid heart disease, n (%)** | 9 (10.6) |
| **Liver metastatic involvement >50%, n (%)** | 38 (44.7) |
| **Extra-hepatic metastases, n (%)** | 79 (92.9) |
| Peritoneum | 44 (51.8) |
| Bones | 52 (61.2) |
| Lung | 14 (16.5) |
| Non-regional lymph nodes | 57 (67.1) |
| **Ki-67 index (%), median (IQR)** (5 missing) | 5 (2-10) |
| **Tumour grade, n (%)** (5 missing) | |
| G1 | 25 (31.3) |
| G2 | 52 (65) |
| G3 | 3 (3.8) |
| **Time from diagnosis of metastatic NET (months), median (IQR)** | 48.1 (23.9-85.8) |
| **Number of previous treatment lines, median (IQR)** | 2 (1-3) |
| **Previous treatment, n (%)** | |
| Primary tumour surgery | 64 (75.3) |
| Liver locoregional treatment | 52 (61.2) |
| Targeted therapies | 23 (27.1) |
| Long-acting somatostatin analogues | 72 (84.7) |
| Systemic chemotherapy | 28 (32.9) |

| | |
|---|---|
| *3 gastrinomas and 2 glucagonomas | |

### PRRT and post-PRRT scintigraphy

Eighty-five PRRT treatments were administered to the 80 patients, including five patients who received two distinct treatments. Among the latter, the second PRRT sequence was initiated after a median of 23.6 months (20-30.8). All PRRT treatments consisted in four cycles, excepted for 13 of them that consisted in two (n=5) or three (n=8) cycles because of early progression (n=7), persistent medullar toxicity (n=4) or clinical worsening (n=2). The average time interval between C1 and C2 was 2.1 months (2.1-2.4). Overall, 253/322 PRRT cycles were administered at full dose and the 69 remaining cycles (21.4%) were administered at reduced dose. This concerned 22 treatments and was due to patient frailty with major bone metastatic involvement (77.2%), and/or altered blood count or liver tests (45% each). Long-acting SSAs were administered concomitantly to PRRT in 58.8% of the cases (with antisecretory or antitumor intent in 47.1% and 11.8% of cases, respectively).

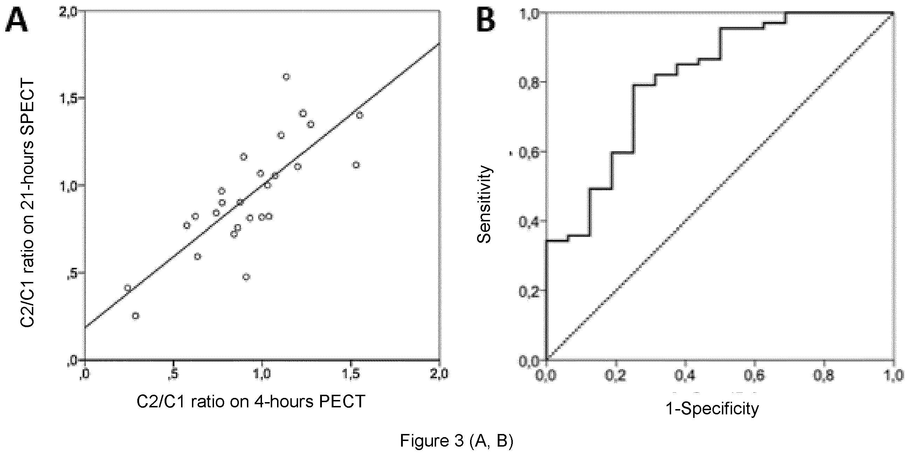

Post-PRRT scintigraphy was performed at 21 hours for 40 treatments (47%), at 4 hours for 19 treatments (22%) and at both acquisitions for 26 treatments (31%). Among the latter, we found a strong correlation between the C2/C1 ratio measured at 21 hours and that measured at 4 hours (rho=0.79; p<0.001) (Figure 3A). Therefore, for all further analyses, we considered one unique C2/C1 ratio per treatment, in priority the 4-hour C2/C1 ratio whenever available, or otherwise the 21-hour ratio.

### Objective response to PRRT and association with the C2/C1 SPECT ratio

Baseline CT-scan was performed 1.4 months (0.5-2.1) before C1, on average. RECIST-defined morphological response to PRRT was evaluable for 83/85 treatments, on CT scans performed, on average, 2.6 months (2.0-3.0) after the last PRRT injection (and 9.1 months (8.6-9.9) after the first PRRT cycle). Overall, the rates of objective response, stability and progression were 19.2% (n=16), 68.7% (n=57) and 12.1% (n=10), respectively.

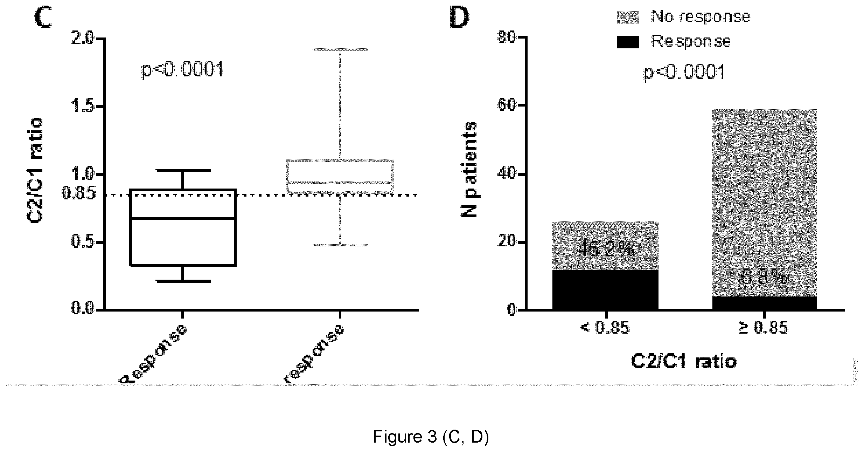

On ROC analysis, the C2/C1 ratio significantly correlated with OR (area under the curve 0.813 ± 0.062, p<0.001) (Figure 3B). The median C2/C1 ratio was 0.67 (0.33-0.89) among responders, and 0.94 (0.87-1.11) among non-responders (p<0.0001) (Figure 3C). A C2/C1 ratio at 0.85 (i.e., corresponding to a 15% decrease between C1 and C2) was the best threshold predicting objective response, with a sensitivity and specificity of 79.1% and 75%, respectively. The objective response rate was 46.2% in case of C2/C1 ratio < 0.85 was 46.2%, compared to 6.8% otherwise (p<0.0001) (Figure 3D). Of note, the geometric mean of the measured count rate at C1 was not significantly associated with objective response (AUC-ROC = 0.619 ± 0.077, p=0.14). At multivariable analysis adjusted for NET primary origin and grade, a C2/C1 ratio < 0.85 remained significantly associated with objective response (OR 6.92, 95% CI [1.64-29.1], p=0.008) (Table 2).

**Table 2. Multivariable analysis of factors associated with the probability of objective response after PRRT**

| | **Multivariate analysis** | | |
|---|---|---|---|
| | **OR** | **95% Cl** | **p** |
| **C2/C1 ratio < 0.85** | 6.92 | 1.64-29.1 | 0.008 |
| **Primary NET (small-intestine NET vs. other)** | 0.12 | 0.02-0.64 | 0.013 |
| **G2/G3 vs. G1** | 3.8 | 0.60-23.4 | 0.156 |

### Progression-free survival

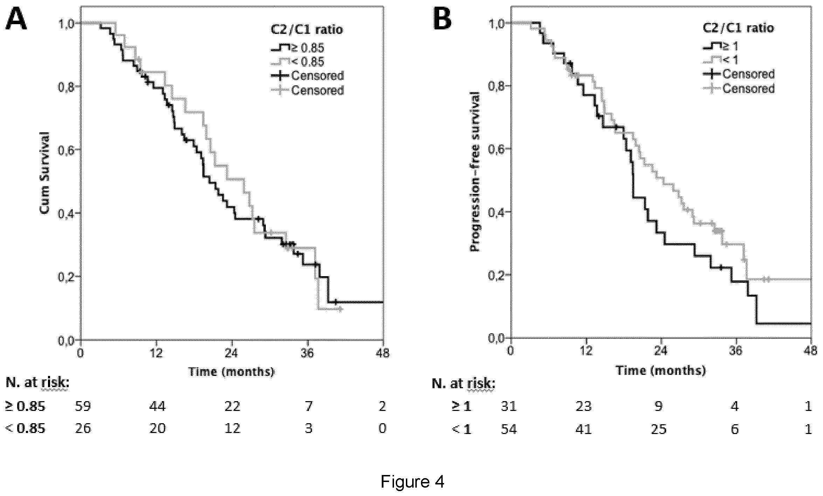

Median PFS was 21.4 months (95% Cl, 17.9-24.9). It was numerically longer in case of C2/C1 ratio < 0.85 (25.9 months, 95% CI [18.6-33.2]; vs. 20.4 months, 95% CI [16.7-24.1]), although the difference was not statistically significant (p=0.84) (Figure 4A). One-, 2- and 3-year PFS rates were 84.4 ± 7.2%, 50.7 ± 10.2% and 29 ± 9.4%, respectively, compared with 79.4 ± 5.3%, 41.9 ± 6.7% and 23.7 ± 6.4%, respectively, in cases with C2/C1 SPECT ratio ≥ 0.85.

When varying the threshold of C2/C1 SPECT ratio at 1, median PFS was still longer in case of ratio < 1 (24.4 months, 95% CI [17.4-31.4]; vs. 19.5 months, 95% Cl [18.9-20.1]; p=0.27) (Figure 4B). One-, 2- and 3-year PFS rates were 83.3 ± 5.1%, 50.8 ± 7.1% and 29.7 ± 7.2%, respectively, compared with 77 ± 7.6%, 33.4 ± 9% and 17.8 ± 7.5%, respectively, in cases with C2/C1 SPECT ratio ≥ 1.

At multivariable analysis adjusted for the number of PRRT cycles, prior chemotherapy administration, the presence of peritoneal metastasis, the Ki-67 index and the concomitant administration of long-acting somatostatin analogues, a C2/C1 ratio < 1 was significantly associated with a decreased risk of progression or death after PRRT (OR 0.52, 95% CI [0.30-0.93], p=0.026) (Table 3).

**Table 3. Multivariable analysis of factors associated with the risk of progression or death after PRRT**

| | **HR** | **95% Cl** | **p-value** |
|---|---|---|---|
| **C2/C1 ratio < 1** | 0.52 | 0.30-0.93 | 0.026 |
| **PRRT cycles < 4 (vs. 4 cycles)** | 4.99 | 2.09-11.95 | <0.001 |
| **Prior chemotherapy (vs. absence)** | 2.89 | 1.32-4.69 | 0.005 |
| **Peritoneal metastases (vs. absence)** | 2.23 | 1.25-3.98 | 0.006 |
| **Ki-67 index (each additional 1%)** | 1.05 | 1.01-1.09 | 0.024 |
| **Concomitant somatostatin analogues (vs. absence)** | 0.64 | 0.35-1.19 | 0.157 |

**Results** The C2/C1 ratio was lower in responders (median 0.67 vs 0.94, p<0.0001). A ratio < 0.85 best predicted tumour response (AUC 0.81, p<0.001), which remained significant in multivariate analysis (OR 6.9, p=0.008). Median PFS was 21 months in the whole cohort, 26 months for a ratio < 0.85 and 20 months for a ratio ≥ 0.85 (p=0.84). In multivariate analyses, a reduction in uptake between C2 and C1 (ratio < 1) was significantly associated with a reduction in the risk of tumour progression (p=0.02).

**Conclusion** The evolution of NET uptake between C1 and C2 is a simple tool for early prediction of PRRT efficacy. This biomarker could help identifying early the patients who may benefit from treatment optimisation or change.

### Discussion

In this study, we report for the first time the possible use of post-PRRT scintigraphy as an early biomarker of PRRT efficacy in patients with advanced NETs. Patients had a higher probability of objective response if ¹⁷⁷Lu uptake at C2 decreased by ≥ 15% compared to C1, and a longer PFS in case of any decrease of uptake. Importantly, this biomarker was measured 2.1 months after PRRT initiation on average, while tumour response was measured on average 9.1 months after PRRT initiation on average. Therefore, this biomarker may help identifying, with a minimal delay, the patients who might benefit from treatment change or optimisation.

The use of post-PRRT scintigraphy has been reported before. It is usually performed following PRRT administration in order to verify the appropriate targeting of NET localisations (Huizing *et al.* 2018 ([5]); Aalbersberg *et al.* 2022 ([6])).

Post-PRRT early scintigraphy (at 4 hours) was previously shown feasible and informative (Mahajan et al. 2019 ([7])): Mahajan S et al. confirmed the similar PRRT distribution between early whole body scintigraphy and whole body scintigraphy at 21h after infusion.

For personalized assessment, it may be also integrated any predictive biomarker for PRRT. Prior PRRT, there are few predictive biomarkers allowing to identify the potentially good responders. A high lesion's SUVmax at pretherapeutic ⁶⁸Ga-DOTATATE PET-CT could be predictive of a good response. The Clinical Score (CS), including 5 clinical elements (available treatments of tumor type outside of PRRT, prior systemic treatments, disease-related symptoms, tumor burden in critical organs and the presence of peritoneal carcinomatosis) predicted the PFS with PRRT. A lower score improved the PFS and suggested to position PRRT in the first line. A positive ¹⁸F-FDG prior PRRT administration is an independent poor prognostic factor for PFS and OS. Futhermore, PPQ is a blood biomarker that predicts PRRT efficacy and NETest can monitoring the response during PRRT . According the two last tools, blood biomarkers isn't available in all centers.

Scintigraphy using radiolabelled somatostatin analogues is necessary prior PRRT because it confirms SSTR expression into NETs' lesions. Unfortunately, most of studied didn't prove the usefulness of these imaging to assess earlier the PRRT response because studies conclusion were contradictory (Gabriel *et al.* 2009 ([8]); Haug ([9])).

Haug et al. ([9]) performed a ⁶⁸Ga-DOTATATE PET/CT after the first cycle. His retrospective study described that patient with a decline in SUV tumour / spleen after finishing the first cycle had a significant longer time to progression (TTP). However, Delta SUVmax did not emerge as a significant predictor. Other studies didn't find the usefulness of PET for assessing therapy responses (Gabriel et al 2009 ([10])). Moreover, study find delta SUVmax fluctuated randomly after cycles.

Our study facilitates the response assessment because we used post-therapeutic scintigraphy. These exams were already acquired after each cure of PRRT without additional imaging method. That's why, it's easy to include them to managed NET's therapy. A decreasing of metastases uptake would maintained PRRT. An increasing of metastasis uptake would be suspected a real disease progression.

Currently, there is no reliable recommendation to assess the tumour response during the eight months of NETs PRRT. However, the early prediction of the response is essential to avoid the side effects and unnecessary infusion of ¹⁷⁷Lu-DOTATATE and decrease the cost.

### Conclusion:

There is a real potential of post-therapeutic scintigraphy to assess PRRT response for NETs. A decrease of uptake on liver metastasis between post-therapeutic C1 and post-therapeutic C2 scintigraphy predicts the tumour response and may play a future role to manage a personalized treatment.

### Reference List

- 1.: Pavel *et al.* 2016, 2020;
- 2.: de Mestier *et al.* 2020
- 3.: Eisenhauer *et al.* 2009
- 4.: Yordanova *et al.* 2018
- 5.: Huizing *et al.* 2018
- 6.: Aalbersberg *et al.* 2022
- 7.: Mahajan et al. 2019
- 8.: Gabriel *et al.* 2009
- 9.: Haug *et al.*
- 10.: Gabriel et al 2009

## Claims

1. An ex *vivo* imagery method for predicting the efficacy of peptide-receptor radionuclide therapy (PRRT) in a patient with a malignant tumor, comprising the steps of:
a) Measuring tumor uptake-after a first cycle (C1) and a second cycle (C2) of PRRT to the patient, and
b) comparing tumor uptake measured at C1 and tumor uptake measured at C2,
Wherein:
• If tumor uptake at C2 is inferior to tumor uptake at C1, the complete PRRT is considered likely to have an efficacy for the patient, or
• If tumor uptake at C2 is superior to tumor uptake at C1, the complete PRRT is considered unlikely to have an efficacy for the patient.

2. A method according to claim 1, wherein the peptide-receptor radionuclide is at least one compound chosen among ¹⁷⁷Lu-DOTA-peptide, for example ¹⁷⁷Lu-DOTATATE or ¹⁷⁷Lu-DOTATOC, ¹⁷⁷Lu-PSMA, ²²⁵Ac-DOTA-peptide and ⁹⁰Y-DOTA-peptide.

3. A method according to any of the preceding claims, wherein the measurement is performed by a nuclear imaging technique.

4. A method according to claim 3, wherein the nuclear imaging technique is scintigraphy.

5. A method according to any of the preceding claims, wherein step a) comprises the steps of:
a1) contouring the whole organ which includes the tumor on nuclear imaging, and
a2) measuring tumor uptake, above threshold of normal organ uptake.

6. Method according to claim 5, wherein step a) comprises also the step of:
a3) calculating the tumor uptake.

7. Method according to claim 5 or 6, wherein step b) comprises the step of calculating the C2/C1 ratio of tumor uptake,
and wherein:
• If the C2/C1 ratio is < 0.85, the complete PRRT is considered likely to have an efficacy for the patient, or
• If the C2/C1 ratio is ≥ 0.85, the complete PRRT is considered unlikely to have an efficacy for the patient.

8. A method according to claim 7, wherein if the C2/C1 ratio < 1.00, the complete PRRT is considered likely to have an efficacy for the patient compared to a patient having a C2/C1 ratio ≥ 1.00.

9. A method according to anyone of the preceding claims, wherein the measurement is performed between about 1 and about 96 hours after PRRT administration.

10. A method according to any of the preceding claims, wherein step a) of measuring the radioactivity is performed on the primary tumour, in metastases or in both.

11. A method according to any of the preceding claims, wherein a complete PRRT treatment comprises at least 2 cycles.

12. A method according to any of the preceding claims, wherein every cycle of PRRT has the same dose of radionuclides.

13. A method according to anyone of the preceding claims, wherein said malignant neoplasm is chosen among neuroendocrine neoplasms, prostate neoplasms, breast neoplasms, liver neoplasms, and pancreatic neoplasms.

14. A method according to claim 13, wherein said neuroendocrine tumour is a metastatic neuroendocrine tumour selected from the group comprising small-bowel or pancreatic, grade 1 neuroendocrine tumor or grade 2 neuroendocrine tumor.
